# EUROPÄISCHE PATENTSCHRIFT

(11) **EP 1 878 456 B1**
(45) Veröffentlichungstag und Bekanntmachung des Hinweises auf die Patenterteilung: **20.08.2014**
(21) Anmeldenummer: 06014498.7
(22) Anmeldetag: 12.07.2006
(51) Int. Cl.: A61M 15/08, B05B 1/28, B05B 1/14, B05B 11/00

(54) **Sprühflasche mit einer Düse für das Applizieren von Nasentropfen in das Innere der Nase**
Spray bottle with a nozzle for delivering nasal drops into the nasal cavity
Flacon pulvérisateur muni d'une buse pour pulvériser des gouttes dans le nez

(43) Veröffentlichungstag der Anmeldung: 16.01.2008
(73) Patentinhaber: Kurz, Hans-Rainer, 26639 Wiesmoor (DE); Krämer, Ulrich, Dr.med., 26129 Oldenburg (DE)
(72) Erfinder: Kurz, Hans-Rainer, 26639 Wiesmoor (DE); Krämer, Ulrich, Dr.med., 26129 Oldenburg (DE)
(74) Vertreter: Jabbusch, Matthias

(56) Entgegenhaltungen:
- EP-A- 1 475 157
- WO-A-99/32185
- WO-A-2004/108197
- DE-A1-102005 004 889
- JP-A- 8 318 184
- US-A- 5 082 149
- US-B1- 6 276 568

## Beschreibung

Die Erfindung betrifft eine Nasensprayflasche, mit einem Flüssigkeitstank und mit zumindest einer mit dem Flüssigkeitstank flüssigkeitsleitend verbundenen Spraydüse zum Sprühen von Nasentropfen in Form eines Sprühnebels in ein Nasenloch, wobei die Spraydüse zumindest ein die Flüssigkeit seitlich zur Längserstreckung der Spraydüse abgebendes Austrittselement aufweist.

Sprayflaschen werden zum Vernebeln einer im Flüssigkeitstank der Sprayflasche enthaltenen Flüssigkeit eingesetzt. Die Sprayflaschen können dabei aus Metall oder auch aus Kunststoff ausgebildet sein, sie können mit einem Druckmittel beaufschlagt sein oder durch ein Zusammendrücken des Flüssigkeitstankes oder auf andere Weise betätigt werden.

Nasensprayflaschen dieser Art werden für das Applizieren von Nasentropfen in das Innere der Nase eingesetzt. Mit Hilfe der Spraydüse kann ein Nasentropfen in ein Nasenloch eingesprüht werden. Die Düse dient dabei zum Überwinden der inneren Nasenkappe.

Bei bekannten Nasensprays ist die Spraydüse regelmäßig kegelförmig ausgebildet. In der Kegelspitze befindet sich ein Durchbruch, durch den der Nasentropfen in Sprühform austreten kann. Wird ein Druck auf den Flüssigkeitstank ausgeübt, so tritt aus der Spraydüse ein Flüssigkeitsnebel aus, der eine etwa kegelförmige Ausbildung hat. Der Sprühnebel hat die Form eines sich konusartig erweiternden Kreises, dessen Durchmesser sich mit zunehmendem Abstand von der Spraydüse vergrößert.

Aufgrund dieser allseitigen Erweiterung ist zwar gewährleistet, daß der versprühte Nasentropfen in dem Nasenflügel verteilt wird, allerdings tritt dabei der Nachteil auf, daß der Nasentropfen auch auf die Nasenscheidewand trifft.

Um einen Schnupfen zu lindern, ist es erforderlich, die laterale Nasenwand mit den dort angeordneten Nasenmuscheln zu besprühen. Das Besprühen der Nasenscheidewand ist dagegen nachteilig, da durch das Sprühmittel die an der Nasenscheidewand angeordneten Schleimhäute beeinträchtigt werden, ihre Dicke sich reduzieren kann und es schließlich sogar zu Beschädigungen in Form von Durchlöcherungen der Nasenscheidewand kommen kann.

In der EP 1 475 157 A1, der WO 2004/108197 A1 sowie der WO 99/32185 sind bereits Nasensprayflaschen vorgeschlagen worden, aus denen Nasentropfen in Sprühform nicht allseitig austreten, sondern in bestimmten Richtungen. Bohrungen in den Düsen dieser Nasensprayflaschen sind definiert angeordnet, jedoch ist auch in diesen Druckschriften keine besondere Maßnahme jeweils offenbart, die Nasenscheidewand von aufgesprühten Nasentropfen freizuhalten.

Der Erfindung liegt die Aufgabe zugrunde, eine Nasensprayflasche der eingangs genannten Gattung aufzuzeigen, die für einen Einsatz als Nasenspray ohne eine Beschädigung der Nasenscheidewand geeignet ist.

Diese Erfindung ist erfindungsgemäß dadurch gelöst, daß dem Austrittselement auf der der Sprührichtung abgewandten Seite ein in das Nasenloch einführbarer Vorsprung zugeordnet ist, der nach Einführen ein Besprühen der Nasenscheidewand durch den Sprühnebel verhindert.

Bei der erfindungsgemäßen Nasensprayflasche wird der austretenden Flüssigkeit eine laterale Sprayrichtung vorgegeben. Dieses Vorgeben wird dadurch erreicht, daß die Spraydüse ein Austrittselement hat, das den Sprühnebel seitlich austreten läßt.

Der gezielt seitlich gerichtete Sprühstrahl kann nun derart in ein Nasenloch eingebracht werden, daß er lateral gegen die Nasenwand mit den dort angeordneten Nasenmuscheln geführt wird. Für die Linderung eines Schnupfenleidens ist insbesondere das Besprühen der unteren Nasenmuschel bzw. des Kopfes der unteren Nasenmuschel erforderlich. Da abweichend von der seitlichen Sprührichtung kein Flüssigkeitsnebel aus der erfindungsgemäßen Sprayflasche austritt, gelangt an die Nasenscheidewand kein Nasentropfen. Die Schleimhäute der Nasenscheidewand werden dadurch geschont und Beschädigungen der Nasenscheidewand zuverlässig verhindert.

Um das gezielte seitliche Abgeben der Flüssigkeit durch die erfindungsgemäße Nasensprayflasche zu unterstützen, sieht die Erfindung vor, daß dem Austrittselement auf der der Sprührichtung abgewandten Seite ein in das Nasenloch einführbarer Vorsprung zugeordnet ist. Dieser Vorsprung dient als Sprühstrahlbarriere in Richtung eines Bereiches, der nicht besprüht werden soll. Der Vorsprung ist so in das Nasenloch einzuführen, daß er der Nasenscheidewand zugeordnet ist. Er verhindert dann ein Besprühen der Nasenscheidewand durch den Sprühnebel.

Der Vorsprung kann dabei eine plattenförmige Ausbildung haben, so daß er mit dieser abgeflachten Bauform der Platte gegen die Nasenscheidewand plan anlegbar ist. Dadurch ist zugleich eine Ausrichtung der Spraydüse in einem Nasenloch gegeben. Beim Besprühen beider Nasenlöcher ist die Sprayflasche beim Umsetzen von einem Nasenloch ins andere Nasenloch um 180° zu drehen, damit in beiden Nasenlöchern der vorzugsweise plattenförmige Vorsprung an der Nasenscheidewand zur Anlage kommt.

Das Austrittselement kann in einfacher Ausbildung eine Bohrung sein. Diese Bohrung ist jedoch nicht in der Spitze einer Spraydüse angeordnet, sondern in einem seitlichen Bereich. Die Spraydüse kann beispielsweise eine etwa kegelförmige Ausbildung haben und das Austrittselement in der Kegelmantelfläche angeordnet sein. Es können auch mehrere Austrittselemente vorgesehen sein, aus allen Austrittselementen austretende Flüssigkeit wird seitlich abgegeben.

Zur weiteren Ausbildung der Erfindung ist noch vorgesehen, daß die Spraydüse in ihrer Außenkontur einen stufenförmigen Absatz hat und daß diesem Absatz ein sich in Richtung des Flüssigkeitstanks erstreckendes Adapterteil zugeordnet ist. Bei bekannten Nasensprays dient der stufenförmige Absatz in der Außenkontur der Spraydüse zum Auflegen von zwei Fingern bzw. Daumen und eines Fingers, um die Spraydüse gegen den Flüssigkeitstank zu führen und dadurch einen Flüssigkeitshebel zu erzeugen. Die aufgelegten Finger können dabei das Einführen der Spraydüse in die Nase verhindern, insbesondere dann, wenn der Abstand zwischen dem stufenförmigen Absatz und der Spitze der Spraydüse gering ist. Die aufgelegten Finger sind dem tiefen Einführen der Spraydüse im Wege. Damit wird bei bekannten Nasensprays häufig die innere Nasenkappe nicht überwunden, so daß nicht sämtlicher ausgestoßener Flüssigkeitsnebel in die Nase gerät.

Durch das vorgesehene Adapterteil wird ermöglicht, daß ein Auslösen der Spraydüse nicht mehr durch Aufbringen einer Kraft direkt auf den stufenförmigen Absatz erfolgt. Die Kraft kann jetzt über das Adapterteil auf den Absatz übertragen werden. Das Adapterteil erstreckt sich dabei in Richtung des Flüssigkeitstanks, es ist also von der Spraydüse weiter entfernt. Dadurch ist es gut greifbar und sind mit dem Adapterteil in Verbindung stehende z. B. Finger einem Einführen der Spraydüse in ein Nasenloch nicht im Wege.

Das Adapterteil ist vorzugsweise ein auf den stufenförmigen Absatz aufgelegter Ring, welcher gleichfalls eine Stufenform aufweist. Die Stufenform des Adapterteils bietet wieder den Finger Auflagemöglichkeiten. Es können auch stufenartig ausgebildete Stege vorgesehen sein.

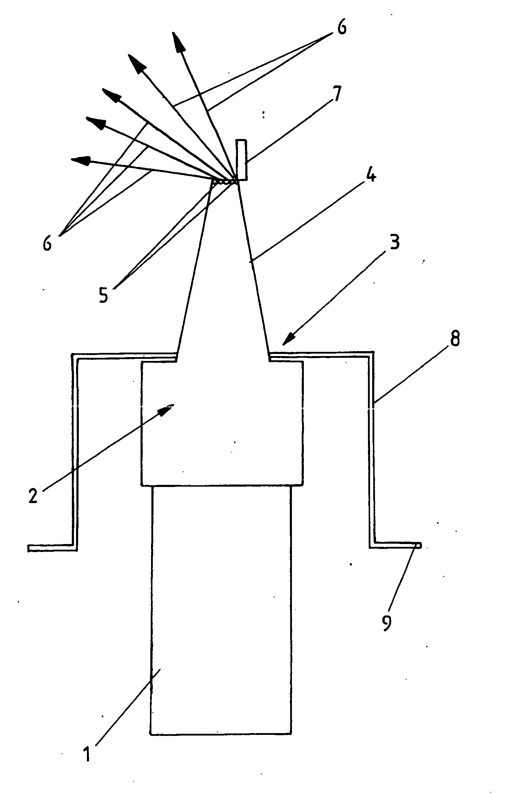

Ein Ausführungsbeispiel der Erfindung, aus dem sich weitere erfinderische Merkmale ergeben, ist in der Zeichnung dargestellt. Die einzige Figur der Zeichnung zeigt eine Seitenansicht einer erfindungsgemäßen Nasensprayflasche.

Die in der Zeichnung dargestellte Nasensprayflasche weist einen Flüssigkeitstank 1 auf, auf den eine Spraydüse 2 aufgesteckt ist. Die Spraydüse 2 sitzt auf einem nicht weiter dargestellten Auslaßventil des Flüssigkeitstanks 1 auf.

Die Spraydüse 2 weist oberhalb eines stufenförmigen Absatzes 3 einen etwa kegelförmigen Abschnitt 4 auf. In der Spitze dieses Abschnittes 4 sind in die Spraydüse 2 mehrere Bohrungen 5 eingebracht. Diese Bohrungen 5 bilden Austrittselemente für im Flüssigkeitstank 1 vorhandene Flüssigkeit in Form eines Sprühnebels aus. Ein austretender Sprühnebel ist durch Pfeile 6 dargestellt. Der Sprühnebel ist bezüglich der Längserstreckung des kegelförmigen Abschnittes 4 der Spraydüse 2 seitlich ausgerichtet. Den Bohrungen 5 ist auf der der Sprührichtung abgewandten Seite noch ein Vorsprung 7 zugeordnet. Dieser Vorsprung 7 weist eine plattenförmige Ausbildung auf.

Auf den stufenförmigen Absatz 3 der Spraydüse 2 ist ein sich in Richtung des Flüssigkeitstanks 1 erstreckendes Adapterteil 8 aufgelegt. Die Auflage kann beispielsweise durch eine Verklebung fixiert sein, auch eine einstückige Ausbildung von Spraydüse 2 und Adapterteil 8 ist möglich. Das Adapterteil 8 weist stufenförmig angesetzte Abschnitte 9 auf.

## Patentansprüche

1. Nasensprayflasche, mit einem Flüssigkeitstank (1) und mit zumindest einer mit dem Flüssigkeitstank (1) flüssigkeitsleitend verbundenen Spraydüse (2) zum Sprühen von Nasentropfen in Form eines Sprühnebels in ein Nasenloch, wobei die Spraydüse (2) zumindest ein die Flüssigkeit seitlich zur Längserstreckung der Spraydüse (2) abgebendes Austrittselement aufweist,
**dadurch gekennzeichnet,**
**dass** dem Austrittselement auf der der Sprührichtung abgewandten Seite ein in das Nasenloch einführbarer Vorsprung (7) zugeordnet ist, der nach Einführen ein Besprühen der Nasenscheidewand durch den Sprühnebel verhindert.

2. Nasensprayflasche nach Anspruch 1, **dadurch gekennzeichnet, daß** das Austrittselement eine Bohrung (5) ist.

3. Nasensprayflasche nach Anspruch 1 oder 2, **dadurch gekennzeichnet, daß** die Spraydüse (2) eine etwa kegelförmige Ausbildung hat und daß das Austrittselement in der Kegelmantelfläche angeordnet ist.

4. Nasensprayflasche nach einem der vorhergehenden Ansprüche, **dadurch gekennzeichnet, daß** der Vorsprung (7) eine plattenförmige Ausbildung hat.

5. Nasensprayflasche nach einem der vorhergehenden Ansprüche, **dadurch gekennzeichnet, daß** die Spraydüse (2) in ihrer Außenkontur einen stufenförmigen Absatz (3) hat und daß diesem Absatz (3) ein sich in Richtung des Flüssigkeitstanks (1) erstreckendes Adapterteil (8) zugeordnet ist.

6. Nasensprayflasche nach Anspruch 5, **dadurch gekennzeichnet, daß** das Adapterteil (8) ein auf den stufenförmigen Absatz (3) aufgelegter Ring ist, welcher gleichfalls eine Stufenform aufweist.

## Claims

1. A nasal spray bottle with a fluid reservoir (1) and with at least one spray nozzle (2), connected in a fluid-transporting manner to the fluid reservoir (1), for spraying nasal drops in the form of an atomised spray into a nostril, wherein the spray nozzle (2) has at least one outlet element discharging the fluid laterally to the longitudinal plane of the spray nozzle (2),
**characterised in**
**that** assigned to the outlet element on the side turned away from the spray device there is a projection (7) that can be inserted into the nostril, which after insertion prevents the nasal septum being sprayed with the atomised spray.

2. The nasal spray bottle according to claim 1, **characterised in that** the outlet element is a bored hole (5).

3. The nasal spray bottle according to claim 1 or 2 **characterised in that** the spray nozzle (2) has a slightly tapered design and **in that** the outlet element is arranged in the tapered lateral surface.

4. The nasal spray bottle according to any one of the preceding claims **characterised in that** the projection (7) has a plate-shaped design.

5. The nasal spray bottle according to any one of the preceding claims **characterised in that** in its outer contour the spray nozzle (2) has a step-shaped heel (3) and that an adapter part (8) extending in the direction of the fluid reservoir (1) is assigned to this heel (3).

6. The nasal spray bottle according to claim 5 **characterised in that** the adapter part (8) is a ring that is placed on the step-shaped heel (3) and also has a stepped shape.

## Revendications

1. Flacon de spray nasal, comprenant un récipient de liquide (1) et au moins une buse de spray (2) reliée en dirigeant le liquide au récipient de liquide (1) pour pulvériser des gouttes nasales sous forme de brouillard pulvérisé dans une narine, sachant que la buse de spray (2) présente au moins un élément de sortie fournissant le liquide sur le côté de l'extension longitudinale de la buse de spray (2),
**caractérisé en ce**
**qu'**une saillie (7) pouvant être introduite dans la narine est attribuée à l'élément de sortie sur le côté détourné du sens de pulvérisation, qui empêche que la cloison nasale ne reçoive la pulvérisation de brouillard pulvérisé après l'introduction.

2. Flacon de spray nasal selon la revendication 1, **caractérisé en ce que** l'élément de sortie est un perçage (5).

3. Flacon de spray nasal selon la revendication 1 ou 2, **caractérisé en ce que** la buse de spray (2) est conçue environ conique et que l'élément de sortie est disposé dans la surface d'enveloppe conique.

4. Flacon de spray nasal selon l'une des revendications précédentes, **caractérisé en ce que** la saillie (7) est en forme de plaque.

5. Flacon de spray nasal selon l'une des revendications précédentes, **caractérisé en ce que** la buse de spray (2) a un épaulement (3) étagé dans son contour extérieur et qu'une pièce adaptatrice (8) s'étendant en direction du récipient de liquide (1) est attribuée à cet épaulement (3).

6. Flacon de spray nasal selon la revendication 5, **caractérisé en ce que** la pièce adaptatrice (8) est une bague placée sur l'épaulement (3) étagé, laquelle présente également une forme étagée.
